# EUROPEAN PATENT SPECIFICATION

(11) **EP 1 056 432 B1**
(45) Date of publication and mention of the grant of the patent: **09.07.2003**
(21) Application number: 98907079.2
(22) Date of filing: 27.02.1998
(51) Int. Cl.: A61K 7/04, A45D 31/00

(54) **IMPROVED CATALYTIC POLYMERIZATION ACCELERATOR SYSTEMS**
VERBESSERTES KATALYTISCHES SYSTEM ZUR POLYMERISATIONSBESCHLEUNIGUNG
ACCELERATEURS AMELIORES DE POLYMERISATION CATALYTIQUE

(43) Date of publication of application: 06.12.2000
(73) Proprietor: Mycone Dental Supply Company, Inc., Cherry Hill, NJ 08002 (US)
(72) Inventor: RANEY, Robert, Newton Square, PA 19073 (US)
(74) Representative: Ruffles, Graham Keith
(86) International application number: GB9800619
(87) International publication number: WO99043288

(56) References cited:
- EP-A- 0 374 824
- EP-A- 0 702 948
- DE-A- 19 614 984
- US-A- 5 098 696
- US-A- 5 523 076

## Description

### Field of the Invention

This invention relates generally to bonding materials for use in artificial nails. More particularly, this invention relates to safe catalysts for curing bonding materials in artificial nails.

### Background of the Invention

In the past, polymers for producing artificial nails have been created which promote fast and strong hardening of the artificial nails when they are applied to the nail bed. Typically, catalysts, initiators and accelerators are used in either chemical polymerization systems or photopolymerization systems to promote fast and strong hardening. Previously, organic peroxides have been used to obtain catalytic action in artificial polymers. For example, benzoylperoxide and toluidines in about 0.25% to 1.0% by weight in the polymer is an effective catalyst. However, certain jurisdictions do not allow the use of benzoylperoxide and toluidine and so it is desired to obtain alternatives to such a catalyst in artificial nails. See, for example, U.S. Patent Nos. 5.523,076 and 5,098,696.

In the dental art, tooth surface treating agents and hardening agents are similarly employed to obtain favorable results for artificial teeth, dentures, and other materials. One highly efficient system for curing dental polymers employs barbituric acids and derivatives thereof as photo and chemical catalysts for dental polymers. Barbituric acid derivatives arc expressed by the following general formula: 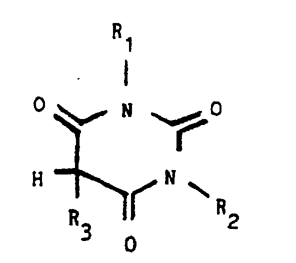 (where R1, R2 and R3 may be the same or different and each may represent an aliphatic, aromatic, alicyclic, or heterocyclic residue or hydrogen atom which may have a substituent, such as a halogen atom, alkyl group, aloxy group, aryl group, or cyclohexyl). Specifically, the following non-limiting list of barbituric acid derivatives is applicable: barbituric acid, 1,3-dimethylbarbituric acid, 1.3-diphenylbarbituric acid, 1,5- dimethylbarbituric acid, 5-butylbarbituric acid, 5-ethylbarbituric acid 5-isopropylbarbituric acid, 5-cyclohexylbarbituric acid, 1,3,5-trimethylbarbituric acid, 1,3-dimethyl-5-ethylbarbituric acid, 1,3-dimethyl-n-butyibarbituric acid, 1,3-dimethyl-5-isobutylbarbituric acid, 1,3-dimethyl-5-tert-butylbarbituric acid, 1,3-dimethyl-5-cyclopentylbarbituric acid, 1,3-dimethyl-5-cyclohexylbarbituric acid, 1,3-dimethyl-5-phenylbarbituric acid,1-cyclohexyl-5-ethylbarbituric acid, 1-benzyl-5-phenylbarbituric acid, and thiobarbituric acids; and salts thereof. These compounds are taught in U.S. Patent No. 5,707,611. The use of barbituric acids in dental applications is also described in U.S. Patents 5,663,214; 4,906,446; and 4,115,346.

It would be desirable to employ catalyst systems which do not display pharmacological activity in making artificial fingernails. This has not heretofore been achieved in the art.

### Summary of the Invention

The aforementioned long-felt needs are met and problems solved by artificial human nail structures provided in accordance with the present invention. The structures preferably comprise polymerized alkyl methacrylate and hydroxyalkyl methacrylate, wherein polymerization of the structure is accomplished by a catalyst of barbituric acid. The structures and compositions of the present invention produce hard, strong and flexible artificial nails without the dangers of pharmacological activity displayed by prior art catalysts.

### Detailed Description of Preferred Embodiments

As described in the aforementioned U.S. Patent Nos. 5,523,076 and 5,098,696 artificial nail structures and compositions for making artificial nail structures are generally comprised of a two-part system. The system consists of a liquid portion or binder, and a powder portion or polymeric powder. The liquid binder consists of a monomeric acrylate or methacrylate ester, and a di-, tri-, or multifunctional acrylate or methacrylate ester, and a tertiary amine accelerator. The powder portion generally comprise a polymeric methacrylate or copolymeric methacrylate and an initiator or catalyst such as a peroxide polymerization initiator, for example benzoyl peroxide.

The tertiary amine accelerator and benzoyl peroxide initiators exhibit pharmacological activity and therefore, it is desired to replace them with a non-pharmacological catalysts. In accordance with the present invention, the catalyst is barbituric acid, as described generally above.

A delivery system is also contemplated in accordance with the present invention. The delivery system preferably makes use of porous or hollow polymers that may contain the catalytic components separately. These materials can adsorb and/or absorb the catalyst components and then be blended into the methylmethacrylate/ethylmethacrylate polymer. This may deliver a more stable catalytic system as well as maintaining desirable bulk appearance and workability qualities of the polymer.

### Example

The inventor of the present subject matter believes that the following prophetic example will produce compositions of particular utility in the artificial fingemail art.

| Liquid Binder | |
|---|---|
| Ethyl methacrylate | 90% |
| Ethylene glycol dimethacrylate | 6.9% |
| 1,3,5-trimethl barbituric acid | 3.1% |
| MEHQ methyl ether of hydroquinone | 10 ppm |

| Polymeric Powder | |
|---|---|
| Poly(ethyl methacrylate) | 98.8% |
| Tert-butyl perbenzoate | 1.0% |
| Iron chloride | 0.2% |

It is expected that the above formulation when combined will polymerize to an artificial fingernail material.

## Claims

1. The process of formation of a methacrylate artificial nail structure from a two-part system comprising a liquid portion and a powder portion, **characterised by** the use of barbituric acid or a derivative thereof as catalyst.

2. The process according to claim 1, wherein the barbituric acid derivative is 1,3,5-trimethylbarbituric acid.

3. The process according to claim 1 or 2, wherein the two-part system comprises a liquid binder which has the following composition:
| Liquid Binder | |
|---|---|
| Ethyl methacrylate | 90% |
| Ethylene glycol dimethacrylate | 6.9% |
| 1,3,5-trimethyl barbituric acid | 3.1% |
| MEHQ | 10 ppm |
and a polymeric powder which has the following composition:
| Polymeric Powder | |
|---|---|
| Poly(ethyl methacrylate) | 98.8% |
| Tert-butyl perbenzoate | 1.0% |
| Iron chloride | 0.2% |

4. An artificial nail structure formed from a two-part acrylate system comprising a liquid binder of a monomeric acrylate or methacrylate ester and a di-,tri- or mult-functional acrylate or methacrylate ester, and a polymeric powder portion of a polymeric methacrylate or a copolymeric methacrylate, using barbituric acid or a derivative thereof as catalyst.

5. An artificial nail structure comprising polymerized alkyl methacrylate and hydorxyalkyl methacrylate wherein polymerization of the structure is accomplished by a catalyst of barbituric acid or a derivative thereof.

## Patentansprüche

1. Verfahren zur Erzeugung einer künstlichen Nagelstruktur aus Methacrylat aus einem Zweikomponentensystem, umfassend einen flüssigen Teil und einen Pulverteil, **gekennzeichnet durch** die Verwendung von Barbitursäure oder einem Derivat davon als Katalysator.

2. Verfahren nach Anspruch 1, bei welchem das Barbitursäure-Derivat 1,3,5-Trimethylbarbitursäure ist.

3. Verfahren nach Anspruch 1 oder 2, bei welchem das Zweikomponentensystem ein flüssiges Bindemittel aufweist, das die folgende Zusammensetzung hat:
| Flüssiges Bindemittel: | |
|---|---|
| Ethylmethacrylat | 90% |
| Ethylenglykoldimethacrylat | 6,9% |
| 1,3,5-Trimethylbarbitursäure | 3,1% |
| MEHQ | 10 ppm |
sowie ein Polymerpulver aufweist, das die folgende Zusammensetzung hat:
| Polymerpulver: | |
|---|---|
| Poly(ethylmethacrylat) | 98,8% |
| *tert*-Butylperbenzoat | 1,0% |
| Eisenchlorid | 0,2% |

4. Künstliche Nagelstruktur, erzeugt aus einem zweikomponentigen Acrylatsystem, aufweisend ein flüssiges Bindemittel aus einem monomeren Acrylat- oder Methacrylatester und einem di-, tri- oder multifunktionellen Acrylat- oder Methacrylatester und einem polymeren Pulverteil eines polymeren Methacrylats oder eines copolymeren Methacrylats unter Verwendung von Barbitursäure oder einem Derivat davon als Katalysator.

5. Künstliche Nagelstruktur, aufweisend polymerisiertes Alkylmethacrylat und Hydroxyalkylmethacrylat, wobei die Polymerisation der Struktur mit Hilfe eines Katalysators aus Babitursäure oder einem Derivat davon erzielt wird.

## Revendications

1. Procédé pour la formation d'une structure d'ongle artificiel de méthacrylate à partir d'un système en deux parties comprenant une partie de liquide et une partie de poudre, **caractérisé par** l'utilisation d'acide barbiturique ou d'un dérivé de celui-ci comme catalyseur.

2. Procédé suivant la revendication 1, dans lequel le dérivé d'acide barbiturique est l'acide 1,3,5-triméthylbarbiturique.

3. Procédé suivant la revendication 1 ou 2, dans lequel le système en deux parties comprend un liant liquide qui possède la composition suivante:
| Liant liquide | |
|---|---|
| Méthacrylate d'éthyle | 90% |
| Diméthacrylate d'éthylèneglycol | 6,9% |
| Acide 1,3,5-triméthylbarbiturique | 3,1% |
| MEHQ | 10 ppm |
et une poudre polymère qui possède la composition suivante:
| Poudre polymère | |
|---|---|
| Poly(méthacrylate d'éthyle) | 98,8% |
| Perbenzoate de tert-butyle | 1,0% |
| Chlorure de fer | 0,2% |

4. Structure d'ongle artificiel formée à partir d'un système d'acrylate en deux parties comprenant un liant liquide d'un ester d'acrylate ou de méthacrylate monomère et d'un ester d'acrylate ou de méthacrylate di-, tri- ou multifonctionnel, et d'une partie de poudre polymère d'un méthacrylate polymère ou d'un méthacrylate copolymère, en utilisant l'acide barbiturique ou un dérivé de celui-ci comme catalyseur.

5. Structure d'ongle artificiel comprenant un méthacrylate d'alkyle et un méthacrylate d'hydroxyalkyle polymérisés, dans laquelle la polymérisation de la structure est accomplie par un catalyseur d'acide barbiturique ou un dérivé de celui-ci.
